# EUROPEAN PATENT APPLICATION

(11) **EP 1 913 904 A1**
(43) Date of publication of application: **23.04.2008**
(21) Application number: 03758133.7
(22) Date of filing: 22.10.2003
(51) Int. Cl.: A61F 2/48

(54) **MAGNETICALLY-ACTUATED ARTIFICIAL INTRAURETHRAL SPHINCTER FOR FEMALE PATIENTS**

(30) Priority: 22.10.2002 ES 200202425
(71) Applicant: LABORATORIOS INDAS, S.A., 28223 Pozuelo de Alarcon (ES)
(72) Inventor: RIVERO RODRIGUEZ, G., Inst. de Magnetismo Aplicado, E-28230 Las Rozas - Madrid (ES); MULTINGER DOMINGUEZ, M.M., Inst. Magnetismo Aplic., E-28230 Las Rozas - Madrid (ES); GARCIA PAEZ, J.M., Clinica Puerta de Hierro, E-28035 Madrid (ES); CARBALLIDO RODRIGUEZ, J., Clinica Puerta de Hierro, E-28035 Madrid (ES); JORGE HERRERO, E., Clinica Puerta de Hierro, E-28035 Madrid (ES); FLORES VIDAL, M.S., Clinica Puerta de Hierro, E-28035 Madrid (ES); OLIVIER GOMEZ, C., Clinica Puerta de Hierro, E-28035 Madrid (ES); MARTIN MAESTRO, M., Clinica Puerta de Hierro, E-28035 Madrid (ES); LAFONT MORGADO, P., Clinica Puerta de Hierro, E-28035 Madrid (ES); LORENZO YUSTOS, H., Clinica Puerta de Hierro, E-28035 Madrid (ES); ORTEGO GARCIA, P., Clinica Puerta de Hierro, E-28035 Madrid (ES)
(74) Representative: Carpintero Lopez, Francisco
(86) International application number: PCT/ES2003/000535
(87) International publication number: WO 2004/037134

(57) **Abstract**

Artificial intraurethral sphincter with magnetic activator for female patients, that consists of a device that is installed in the female urethra, in a similar way to a urinary catheter, which gives the patient voluntary control of micturition. Said control is effected by means of an external magnet (6) that is suitably positioned outside the body, which opens a magnetic valve that is incorporated in the invention. When said external magnet (6) is withdrawn, the magnetic valve closes automatically, preventing urine leakage. This valve performs an axial movement of a soft ferromagnetic plunger, activated by the external magnet, opening the orifice so that the urine can be voided. In its normal position, i.e., without activating the exterior magnet, the plunger is attracted by a small internal magnet (5), thus closing the voiding orifice.

## Description

### OBJECT OF THE INVENTION

This descriptive report refers to an application for an Invention Patent corresponding to an artificial intraurethral sphincter with magnetic activator for female patients. It consists of a device that is installed in the female urethra, in a similar way to a urinary catheter, which gives the patient voluntary control of micturition. Said control is effected by means of a magnet that is suitably positioned outside the body, which opens a magnetic valve that is incorporated in the object of the invention. When said magnet is withdrawn, the magnetic valve closes automatically, preventing urine leakage.

### FIELD OF THE INVENTION

This invention is of application in the industry that deals with the manufacture of apparatuses, devices and auxiliary elements for medicine and surgery.

### RECORDS OF THE INVENTION

With regard to other records of the invention, the applicant only knows of the current existence of a magnetic valve described in Invention Patent ES 9900284 applied for in Spain under the aforementioned number, regarding an externally activated magnetic valve for an artificial intraurethral urinary sphincter, applied for on behalf of Madrid Complutense University.

It has been verified that the invention in question should be provided with a miniaturisation of the valve, and the incorporation of a funnel in soft ferromagnetic material with similar characteristics to FeSi, and also in order to open the valve, the position of the external magnetic should be changed from the pubic zone to the perineal zone, reducing its weight.

However, to date, the applicant does not know of the existence at present of an invention that has the same ideal characteristics that have been described above.

### DESCRIPTION OF THE INVENTION

The artificial intraurethral sphincter with magnetic activator for female patients proposed in this invention is itself a clear novelty in its specific field of application.

More specifically, the artificial intraurethral sphincter with magnetic activator for female patients is made in such a way that in addition to the magnetic valve, there are the necessary elements so that the same can be implanted, fixed and removed in a non-invasive form for the patient, and it also presents a series of improvements on the magnetic valve.

In general, the artificial intraurethral sphincter with magnetic activator for female patients consists of an internal fixation element, the purpose of which is to prevent the device that acts as an artificial sphincter from migrating out of the patient's body. It should be indicated that said element is similar to the balloon end of a urinary catheter and it is provided with a balloon that is inflated, after positioning in the bladder, by injecting physiological saline through a catheter incorporated in the device.

As a central point in the design to prevent migration of the device towards the bladder, the invention also has a soft supplement attached to the opposite end of the valve from the bladder, and is provided with a flower petal design situated precisely at the urethral orifice, thus preventing migration of the device towards the bladder.

Said petals are protected by the labium minora and majora, and present a substantially lower probability of infection than infection caused by a urinary catheter.

The design of this element has been revised in such a way that it also acts as a limit to valve plunger movement when it is opened.

The invention has a system for implanting and removing the device, since the implant is effected in a non-invasive form for the patient, similar to the implantation of a urinary catheter. In this case the catheter for filling the bladder balloon is sealed through heat application to the same.

The invention is withdrawn by cutting the heat-sealed end of the catheter and emptying the bladder balloon.

It should be indicated that the relation between the distance between the internal magnet - plunger and the threshold pressure for automatic opening of the valve has been established, and a manufacturing and assembly process has been designed in order to modify said distance, thus adjusting the safety threshold pressure for different patients. For this purpose, the valve design has been modified in order to permit the introduction of ring-shaped supplements of variable thickness between the internal magnet and the sealed O-ring.

The purpose of said supplements is to modify the strength of the internal magnet-plunger that keeps the device closed, thus modifying the corresponding safety threshold pressure.

Likewise, the valve body design has been modified, incorporating a channel that serves as a guide in the same in order to house the catheter that fills and empties the bladder balloon.

To summarise, the invention includes miniaturisation of the valve, and based upon this, the exterior dimensions of the valve have been made, with an exterior diameter of 7.5 millimetres and a length of 25 to 30 millimetres, maintaining sufficient flow so that the bladder is voided in a reasonable time. This is a very important aspect from the point of view of the patient's tolerance of the implant, and implies a corresponding reduction of all elements involved.

A soft ferromagnetic plunger has also been incorporated in the invention, with similar characteristics to FeSi, that has the additional property of being biocompatible, thus avoiding having to coat it with a biocompatible material as is the case of the valve in the aforementioned patent described in the section on records of the invention.

The position of the external magnet for opening the valve has also been changed from the pubic zone to the perineal zone, and this has permitted the weight and volume of the external magnet to be reduced by 60%.

### DESCRIPTION OF DRAWINGS

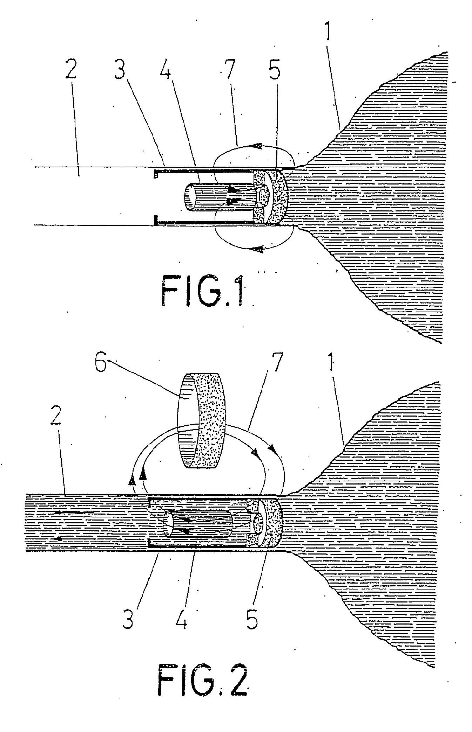

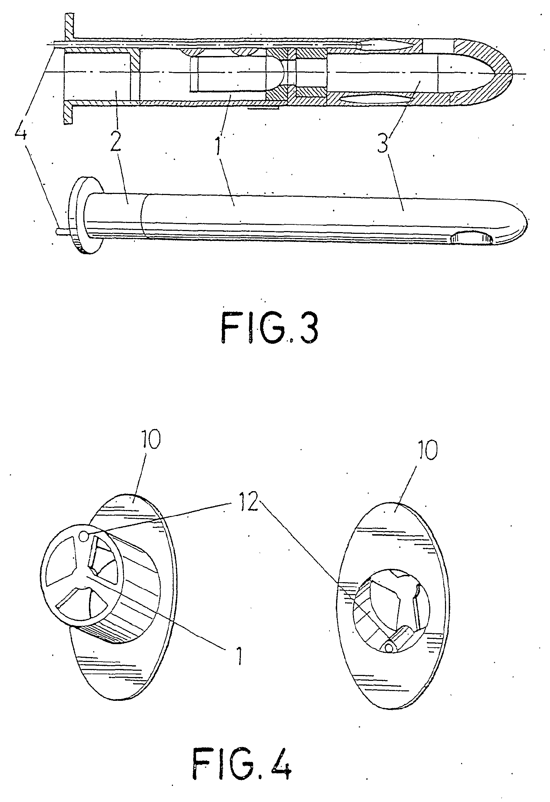

To complement the description of the invention and in order to assist in the full comprehension of the invention's characteristics, this descriptive report is accompanied by a set of diagrams which are of an illustrative and non-restrictive nature and form an integral part of said report, as follows:
Figure number 1. This shows a side view of the object of the invention corresponding to an artificial intraurethral sphincter with magnetic activator for female patients.
Figure number 2. This corresponds to a view of the object shown in figure 1 with the addition of the external magnet.
Figure number 3. This shows a diagram of the side view of the artificial sphincter.
Figure number 4. This shows the external fixation element.

### PREFERENTIAL PERFORMANCE OF THE INVENTION

In view of these figures, and in particular numbers 1 and 2, it can be observed how the artificial intraurethral sphincter with magnetic activator for female patients consists of implanting the following in the bladder neck (1), and specifically in the urethra (2): a valve body (3) that has a plunger (4), an internal magnet (5), and external magnet (6). It is activated as shown by the magnetic field lines (7) in the diagram. Figure number 4 shows the presence of the external fixation element (10) that limits (11) the plunger movement (4), and an orifice (12) for the catheter.

In short, the invention consists of an internal fixation element, the purpose of which is to prevent the device from migrating out of the patient's body. This element is similar to the balloon end of a urinary catheter, and it is provided with a balloon that is inflated, after positioning in the bladder, by injecting physiological saline through a catheter incorporated in the device- This element has a similar form to a soft supplement attached to the opposite end of the valve from the bladder, and is provided with a flower petal design situated precisely at the urethral orifice, thus preventing migration of the device towards the bladder. The petals are protected by the labium minora and majora, and present a substantially lower probability of infection than infection caused by a urinary catheter. The design of this element has been effected in such a way that it also acts as a limit to valve plunger movement when it is opened.

The implant is effected in a non-invasive form for the patient, similar to the implantation of a urinary catheter. In this case the catheter for filling the bladder balloon is sealed through heat application to the same.

The invention is withdrawn by cutting the heat-sealed end of the catheter and emptying the bladder balloon. It should be indicated that the valve body incorporates a channel that serves as a guide in the same in order to house the catheter that fills and empties the bladder balloon.

With regard to the system for adjusting the safety threshold pressure, it should be indicated that the relation between the distance between the internal magnet - plunger and the threshold pressure for automatic opening of the valve has been established, and a manufacturing and assembly process has been designed in order to modify said distance, thus adjusting the safety threshold pressure for different patients.

The valve design has also been modified in order to permit the introduction of ring-shaped supplements of variable thickness between the internal magnet and the sealed O-ring. The purpose of said supplements is to modify the strength of the internal magnet-plunger that keeps the device closed, thus modifying the corresponding safety threshold pressure.

The invention uses a plunger of biocompatible ferromagnetic material, that does not require any external coating after manufacture.

## Claims

1. Artificial intraurethral sphincter with magnetic activator for female patients, designed for implantation in the female urethra, in a similar way to a urinary catheter, giving the patient voluntary control of micturition. It is **characterised by** the following: a valve body (3) that is installed in the urethra (2) and reaches the bladder (1), incorporating a plunger (4), an internal magnet, and an external fixation element (10) that limits (11) plunger movement, and an orifice (12) for the catheter. Micturition is controlled by means of external magnet (6), which activates a magnetic valve that closes automatically when the external magnet (6) is removed. It acts through the axial movement of a soft ferromagnetic plunger, activated by the external magnet (6), opening the orifice so that the urine can be voided.

2. Artificial intraurethral sphincter with magnetic activator for female patients, as described in claim one, **characterised** because in a normal or resting position, when the external magnet is not activated, the plunger (4) is attracted by the internal magnet (5) and the voiding orifice is closed.

3. Artificial intraurethral sphincter with magnetic activator for female patients, as described in claim one, **characterised by** a balloon that is positioned in the bladder and is then inflated by injecting physiological saline through a catheter.

4. Artificial intraurethral sphincter with magnetic activator for female patients, as described in claim one, **characterised by** the external fixation element that is made from a soft supplement attached to the opposite end of the valve from the bladder (1), and is provided with a flower petal design situated at the urethral orifice, acting as a limit to valve plunger movement when it is opened.

5. Artificial intraurethral sphincter with magnetic activator for female patients, as described in the foregoing claims, **characterised** because the catheter for filling the bladder balloon is heat-sealed, and the catheter is removed by cutting the heat-sealed end of the catheter and emptying the bladder balloon.

6. Artificial intraurethral sphincter with magnetic activator for female patients, as described in the foregoing claims, **characterised by** the incorporation of a sealed O-ring, with ring-shaped supplements of variable thickness, located beside the internal magnet (5).

7. Artificial intraurethral sphincter with magnetic activator for female patients, as described in the foregoing claims, **characterised** because the strength of the internal magnet - plunger (6) - (4), that keeps the sphincter closed, modifies the safety threshold pressure.

8. Artificial intraurethral sphincter with magnetic activator for female patients, as described in the foregoing claims, **characterised** because the guide channel houses the catheter that fills and empties the bladder balloon.

9. Artificial intraurethral sphincter with magnetic activator for female patients, as described in the foregoing claims, **characterised** because the plunger is made with a soft, biocompatible, ferromagnetic material with similar characteristics to FeSi, and it is not coated.
